# EUROPEAN PATENT APPLICATION

(11) **EP 1 402 892 A1**
(43) Date of publication of application: **31.03.2004**
(21) Application number: 02733465.5
(22) Date of filing: 11.06.2002
(51) Int. Cl.: A61K 31/505, A61P 11/00, A61P 11/06, A61P 11/14, A61P 29/00, A61P 37/08, C07D 239/557

(54) **ANTIINFLAMMATORIC AND ANTITUSSIVE COMPOSITIONS**

(30) Priority: 11.06.2001 JP 2001175929
(71) Applicant: KYOWA HAKKO KOGYO CO., LTD., Chiyoda-ku, Tokyo 100-8185 (JP)
(72) Inventor: FURUKAWA, Satoru, c/o Nutri-Quest, Chesterfield, MO 63017 (US); AKUTA, Kaori, c/o Kyowa Hakko Kogyo Co., Ltd, Sunto-gun, Shizuoka 411-8731 (JP); ICHIKAWA, Shunji, c/o Kyowa Hakko Kogyo Co., Ltd, Sunto-gun, Shizuoka 411-8731 (JP); MANABE, Haruhiko, c/o Kyowa Hakko Kogyo Co., Ltd, Sunto-gun, Shizuoka 411-8731 (JP)
(74) Representative: Williams, Aylsa
(86) International application number: PCT/JP2002/005789
(87) International publication number: WO 2002/100409

(57) **Abstract**

The object of the present invention is to provide antiinflammatory agents for airway, foods and drinks, animal feeds, food additives, feed additives and a method for preventing or treating airway inflammations. The object is achieved by providing antiinflammatory agents for airway, foods and drinks, animal feeds, food additives and feed additives, each comprising, as the active ingredient thereof, orotic acid or its derivative or a salt thereof; and a method for preventing or treating airway inflammations which comprises administering orotic acid or its derivative or a salt thereof.

## Description

### TECHNICAL FIELD

The present invention relates to antiinflammatory agents for airway, foods and drinks, animal feeds, food additives and feed additives, each comprising, as the active ingredient thereof, orotic acid or its derivative or a salt thereof; and to a method for preventing or treating airway inflammations that comprises administering orotic acid or its derivative or a salt thereof.

### BACKGROUND ART

Allergic diseases such as bronchitis, asthma and pollinosis that are caused by atmospheric pollution are increasing these days, and compositions capable of preventing or relieving airway inflammations caused by these diseases are desired.

Orotic acid is an intermediate in nucleic acid pyrimidine biosynthesis, and it is synthesized from glutamic acid and is converted into uridine monophosphate.

Regarding its pharmaceutical effects, it is known that orotic acid is effective for hepatic diseases and has an immunopotentiating activity and is also effective for ischemic diseases [*Jpn. J. Pharmacology,* 76, 441-444 (1998)], but no one knows its effect for inhibiting airway inflammations.

For antiinflammatory agents for airway, known are steroid inhalants, β-agonists, anticholinergic agents, xanthine derivatives, antiallergic agents, and codeine derivatives. These have side effects, and are therefore unfavorable for supplements or OTC medicines that are generally in common use.

### DISCLOSURE OF THE INVENTION

An object of the present invention is to provide antiinflammatory agents for airway, foods and drinks, animal feeds, food additives and feed additives, each comprising, as the active ingredient thereof, orotic acid or its derivative or a salt thereof; and a method for preventing or treating airway inflammations that comprises administering orotic acid or its derivative or a salt thereof.

The present inventors have assiduously studied various substances, using animal models that reflect allergic asthmatic attack, and, as a result, have found that orotic acid, its derivatives and salts thereof are effective for inhibiting airway inflammations and have completed the present invention.

Specifically, the present invention relates to the following (1) to (7):
(1) Anantiinflammatory agent forairway, which comprises, as the active ingredient thereof, orotic acid or its derivative or a salt thereof.
(2) The antiinflammatory agent for airway of above (1), which has an antitussive effect.
(3) The antiinflammatory agent for airway of above (1) or (2) , which is against airway inflammations selected from those caused by allergic asthma, those caused by pollinosis and those caused by bronchitis.
(4) A food and drink or an animal feed, which comprises orotic acid or its derivative or a salt thereof.
(5) A food additive or a feed additive, which comprises orotic acid or its derivative or a salt thereof.
(6) A method for preventing or treating human or non-human animal airway inflammations, which comprises administering orotic acid or its derivative or a salt thereof.
(7) Use of orotic acid or its derivative or a salt thereof for the manufacture of antiinflammatory agents for airway.

### (a) Antiinflammatory agents for airway comprising, as the active ingredient thereof, orotic acid or its derivative or a salt thereof:

Orotic acid (uracil-4-carboxylic acid), its derivatives and salts thereof in the present invention may be any ones, including, for example, those derived from microorganisms, those obtained through chemical synthesis and those available on the market.

Orotic acid derivatives include, for example, 4-ester derivatives where 4-carboxylic acid is esterified with an alkyl group having from 1 to 6 carbon atoms such as methyl, ethyl, butyl, propyl, pentyl or hexyl; and other derivatives where at least one hydrogen at 1, 2, 3 and 6-positions is substituted with an alkyl group having from 1 to 6 carbon atoms such as methyl, ethyl, butyl, propyl, pentyl or hexyl, or with an alkoxy group having an alkyl group with from 1 to 6 carbon atoms such as methyl, ethyl, butyl, propyl, pentyl or hexyl.

Salts of orotic acid or its derivatives include, for example, alkali metal salts such as sodium salts, potassium salts; alkaline earth metal salts such as magnesium salts, calcium salts; heavy metal salts such as zinc salts; ammonium salts such as ammonium or tetramethylammonium; and organic amine addition salts with morpholine or piperidine.

The antiinflammatory agent for airway of the present invention, which comprises, as the active ingredient thereof, orotic acid or its derivative or a salt thereof is effective for inhibiting inflammations of airway, especially upper airway of cases of asthma such as allergic asthma or cases of allergic disease such as pollinosis or bronchitis, and for relieving symptoms such as coughing caused by these inflammations, and this is therefore useful for preventives and treatments for these diseases.

The antiinflammatory agent for airway of the present invention may contain any other active ingredient effective for preventing, inhibiting or curing airway inflammations (this is hereinafter simply referred to as "other active ingredient"), in addition to orotic acid or its derivative or a salt thereof. The other active ingredient includes, for example , herbs, menthol, antihistaminic agents, and allergy mediator inhibitors.

The antiinflammatory agent for airway of the present invention may be produced in any method well known in the technical field of pharmaceutics, for example, by mixing orotic acid or its derivative or a salt thereof and optionally the above other active ingredient along with one or more pharmaceutically-acceptable carriers.

The administration route of the antiinflammatory agent for airway of the present invention is preferably so selected that it is effective for prevention, inhibition and treatment of airway inflammations, including, for example, oral administration or parenteral administration such as intravenous administration.

Regarding its administration morphology, for example, the agent may be in any form of tablets, capsules, injections, drips, syrups, sublingual tablets, various creams, and suppositories.

Liquid preparations such as syrups that are suitable for oral administration may be produced by the use of diluents, for example, water, saccharides suchas sucrose, sorbitol, fructose; glycols such as polyethylene glycol, propylene glycol; or oils such as sesame oil, olive oil, soybean oil; antiseptics such as p-hydroxybenzoates; and flavors such as strawberry flavor, peppermint. Tablets, powders and granules may be produced by the use of vehicles such as lactose, glucose, sucrose, mannitol; disintegrators such as starch, sodium alginate; lubricants such as magnesium stearate, talc; binders such as polyvinyl alcohol, hydroxypropyl cellulose, gelatin; surfactants such as fatty acid esters; and plasticizers such as glycerin.

Preparations suitable for parenteral administration are preferably germ-free, active ingredient-containing aqueous preparations that are isotonic to the receiver's blood. For example, injections may be prepared by the use of carrier solutions for injections, such as salt solutions, glucose solutions, or mixtures of salt water and glucose solutions.

To the parenteral preparations, at least one ingredient selected diluents, antiseptics, flavors, vehicles, disintegrators, lubricants, binders, surfactants and plasticizers such as those mentioned hereinabove for oral preparations may be added.

The content of orotic acid or its derivative or a salt thereof in the antiinflammatory agent for airway of the present invention is preferably from 1 to 1000 mg, more preferably from 10 to 500 mg, even more preferably from 100 to 200 mg per gram of the medicine of the present invention.

The administration amount and the administration frequency of the antiinflammatory agent for airway of the present invention vary, depending on the administration morphology and on the age, the body weight and the symptom of the case to which it is administered. In oral administration thereof, in general, preferably from 1 to 5000 mg, more preferably from 10 to 1000 mg, even more preferably from 100 to 300 mg of the agent is administered once or a few times a day.

In parenteral administration, for example, in intravenous administration, preferably from 0.5 to 5000 mg/adult, more preferably from 5 to 1000 mg/adult, even more preferably from 50 to 300 mg/adult is administered once or a few times a day.

Daily administration of the antiinflammatory agent for airway of the present invention is effective for preventing airway inflammations.

Daily administration of the antiinflammatory agent for airway of the present invention to cases already having airway inflammations makes it possible to inhibit or cure the airway inflammations and symptoms such as coughing. The administration period is generally from 1 week to 10 years, preferably from 1 month to 5 years.

The antiinflammatory agent for airway of the present invention is applicable not only to humans but also to animals except humans (hereinafter referred to as "non-human animals") . The administration amount to non-human animals is preferably from 0.02 to 100 mg/kg, more preferably from 0.2 to 20 mg/kg, even more preferably from 2 to 10 mg/kg in terms of orotic acid or its derivative or a salt thereof.

### (b) Foods and drinks, animal feeds, food additives or feed additives comprising orotic acid or its derivative or a salt thereof:

The foods and drinks comprising orotic acid or its derivative or a salt thereof are those obtained by adding orotic acid or its derivative or a salt thereof to foods or drinks.

Foods and drinks obtained by adding the food additive of the present invention that will be describedhereinunder to foods and drinks are also within the scope of the foods and drinks of the present invention.

Foods and drinks obtained by adding orotic acid or its derivative or a salt thereof along with the other active ingredient mentioned in the section (a) to foods and drinks are also within the scope of the foods and drinks of the present invention.

The foods and drinks of the present invention may be produced in any method of producing ordinary foods and drinks except that orotic acid or its derivative or a salt thereof and optionally any other active ingredient are added thereto.

For example, the foods and drinks of the present invention that are in the form of drinks or tablets may be produced by optionally adding any other ingredient and additives to orotic acid or its derivative or a salt thereof followed by dissolving or dispersing it in a suitable amount of water or tabletting it. Sweets such as caramels, drops, chocolates, jellies, biscuits and cookies may be produced in an ordinary manner, for example, by mixing orotic acid or its derivative or a salt thereof optionally with any other active ingredient or additives and further optionally with a suitable carrier such as wheat flour, rice flour, starch, corn starch and soybeans, followed by shaping it into suitable forms.

The foods and drinks of the present invention may be produced in any method of granulation such as fluidized bed granulation, stirring granulation, extrusion granulation, rotary granulation, aerial granulation, compression molding granulation, grinding granulation, spraying granulation, jet granulation; or coating such as pan coating, fluidized bed coating, dry coating; or swelling such as puff drying, excess water vaporization, foam mat drying, microwave heating; or extrusion by the use of extrusion granulator or extruder.

The foods and drinks of the present invention may be in anyformof juices, refreshing drinks, teas, lactic acid bacteria drinks, milk products such as fermented milks, cold sweets, butter, cheese, yogurt, processed milks, defatted milks; meat products such as hams, sausages, hamburgers; fish products such as boiled fish pastes, tube-shaped fish paste cakes, fried fish balls; egg products such as seasoned omelets, steamed egg custards; sweets such as cookies, jellies, chewinggums, candies, snacks; breads, noodles, pickles, smoked products, dried fishes, foods boiled down in soy, salted foods, soups, seasonings, etc.

In addition, the foods and drinks of the present invention may be in any other form of, for example, powdery foods, sheet-like foods, bottled foods, canned foods, retorted foods, capsule foods, tablet foods, fluid foods, health drinks, etc.

The foods and drinks of the present invention may be used as health foods or functional foods effective for preventing, inhibiting or curing airway inflammations.

The food additives of the present invention may be prepared in the same manner as that mentioned in the above (a) for oral preparations. Optionally mixed or dissolved with any other food additive, the food additives of the present invention are generally processed into various forms of, for example, powders, granules, pellets, tablets or liquids.

Food additives that are generally used in foods and drinks may be added to the foods and drinks and the food additives of the present invention. For example, they include sweeteners, colorants, preservatives, viscosity stabilizers, antioxidants, coloring matters, bleaching agents, antifungal agents, gumbases, bitters, enzymes, brighteners, souring matters, seasonings, emulsifiers, tonics, formulation matters, flavorings, spice extracts.

The content of orotic acid or its derivative or a salt thereof as well as that of any other active ingredient in the foods and drinks of the present invention varies depending on the morphology of the foods and drinks. In general, the intake of orotic acid or its derivative or a salt thereof from the foods and drinks is preferably from 1 to 5000 mg/adult/day, more preferably from 10 to 1000 mg/adult/day, even more preferably from 100 to 500 mg/adult/day.

The food additives of the present invention are added to foods and drinks to such a degree that the intake of orotic acid or its derivative or a salt thereof from the food or and drink that contains the food additive may fall within the range as above. The foods and drinks may be taken once or a few times a day.

Taking the food or drink of the present invention on a daily basis makes it possible to prevent airway inflammations.

In case of those having airway inflammations, by taking the food or drink of the present invention on a daily basis, their airway inflammatory or coughing symptoms may be inhibited or cured. The intake period is generally from 1 day to 10 years, preferably from 1 month to 5 years.

The animal feeds of the present invention contain orotic acid or its derivative or a salt thereof. The animal feeds that further contain the other active ingredient as in the above (a) are also within the scope of the animal feeds of the present invention.

Animal feeds obtained by adding the feed additive of the present invention mentioned below to animal feeds are also within the scope of the animal feeds of the present invention.

The animal feeds of the present invention may be produced in any method of producing ordinary animal feeds, except that orotic acid or its derivative or a salt thereof and optionally any other active ingredient are added thereto.

In producing the animal feeds of the present invention, orotic acid or its derivative or a salt thereof may be added to any feeds for animals except humans. For example, they include pet feeds for dogs, cats, rats, and feeds for farm animals such as cows, pigs.

The animal feeds of the present invention may be used as health feeds for animals that are effective for preventing, inhibiting or curing airway inflammations.

Animal feeds to which orotic acid or its derivative or a salt thereof is added include, for example, cereals, cereal refuses, vegetable oil refuses, animals-derived feeds, other feeds, purified products, and their mixtures.

The cereals are, for example, milo, wheat, barley, oats, rye, unpolishedrice, buckwheat, foxtailmillet, Chinesemillet, Japanese millet, corn, soybean.

The cereal refuses are, for example, rice bran, defatted rice bran, wheat bran, meal, wheat, germs, barley bran, pellets, corn bran, corn germs.

The vegetable oil refuses are, for example, soybean oil refuse, soybean f lour, linseed oil refuse, cotton seed oil refuse, peanut oil refuse, safflower oil refuse, coconut oil refuse, palm oil refuse, sesame oil refuse, sunflower oil refuse, rapeseed oil refuse, kapok oil refuse, leaf mustard oil refuse.

The animals-derived feeds are, for example, fish meals such as north-sea meal, imported meal, whole meal, coastal meal; and fish solubles, meat meal, meat-bone meal, blood meal, degraded hair, bone meal, side products processed for farm animals, feather meal, silkworm pupae, skim milk, casein, dry whey.

The other feeds are, for example, vegetable stems and leaves such as those of alfalfa, hey cubes, alfalfa leaf meal, false acacia meal; corn side products such as corn gluten, meal, cornglutenfeed, corn steep liquor; starch and processed starch; fermented products such as yeasts, beer refuses, malt roots, alcohol refuse, soy sauce refuse; farm side products such as citrus fruit refuse, bean-curd refuse, coffee refuse, cocoa refuse; as well as cassava, broad beans, guar meal, seaweed, krill, spirulina, chlorella, minerals.

The purified products are, for example, proteins such as casein, albumin; amino acids, starch, cellulose, saccharides such as sucrose and glucose; minerals, vitamins.

The animal feeds of the present invention may be produced in any method of granulation such as fluidized bed granulation, stirring granulation, extrusion granulation, rotary granulation, aerial granulation, compression molding granulation, grinding granulation, spraying granulation, jet granulation; or coating such as pan coating, fluidized bed coating, dry coating; or swelling such as puff drying, excess water vaporization, foam mat drying, microwave heating; or extrusion by the use of extrusion granulator or extruder.

The feed additives of the present invention may be prepared in the same manner as that mentioned in the above (1) for oral preparations. Optionally mixed or dissolved with any other feed additive, the feed additives of the present invention are generally processed into various forms of, for example, powders, granules, pellets, tablets or liquids.

The content of orotic acid or its derivative, or a salt thereof as well as that of any other active ingredient in the animal feeds of the present invention varies depending on the intake condition, the type of the non-human animals to be fed with the animal feeds, and the age and the body weight of the non-human animals. In general, the intake of orotic acid or its derivative or a salt thereof from the animal feeds is preferably from 0.02 to 100 mg/kg animal/day, more preferably from 0.2 to 20 mg/kg animal/day, even more preferably from 2 to 10 mg/kg animal/day.

The feed additives of the present invention are added to animal feeds to such a degree that the intake of orotic acid or its derivative or a salt thereof from the feed that contains the feed additive may fall within the range as above.

Non-human animals to which the present invention is directed are fed with the animal feed of the present invention once or a few times a day. The feed additive of the present invention may be added to oral compositions for airway antiinflammation for non-human animals. The intake amount and the intake period of the antiinflammatory oral compositions for non-human animals may be the same as those of the animal feeds mentioned hereinabove.

Having non-human animals take the animal feed of the present invention on a daily basis makes it possible to prevent airway inflammations.

In case of those having airway inflammations, by having non-human animals take the animal feed of the present invention on a daily basis, their airway inflammatory or coughing symptoms may be inhibited or cured.

Test Examples mentioned below are to confirm the effect of the present invention, concretely demonstrating the ability of orotic acid and zinc orotate to prevent and inhibit the asthmatic attack in allergic asthmatic models.

### Test Example 1 - Effect to airway hypersensitive reaction:

This was to confirm the presence or absence of antiasthmatic effect of orotic acid and zinc orotate. Concretely, the effect of orotic acid and zinc orotate against leukotriene D4 (hereinafter abbreviated to LTD4) - induced airway hypersensitive reaction was investigated in the manner described below.

Expression of LTD4-induced airway hypersensitiveness was carried out according to the method of Sato et al [*Clinical and Experimental Allergy,* 26, 957-963 (1996)], as shown below:

Hartley male guinea pigs (from Nippon SLC) were raised at a constant temperature (22 to 23°C) and a constant humidity (50 to 60 %) for 2 weeks, and healthy animals of those having a body weight of from 150 to 250 g were used in the test. The guinea pigs were anesthetized with urethane (1.2 g/kg) and subj ected to tracheotomy and cannulation. The tracheal cannula was connected to an artificial respiration device for small animals (Model 7025, UGO BASIL by VARASE), and artificial respiration (breathing rate, 60/min; 10 ml/kg) was performed. As an airway contraction index thereof, the pulmonary inflation pressure (hereinafter abbreviated to PIS, its unit being cmH₂O) of the test animal was measuredvia a pressure transducer (TP-603, by Nippon Kodensha). In addition, a cannula was inserted into the carotid duct of the test animal, and the blood pressure through it was also measured via a pressure transducer (Life Kit DX-360, by Nippon Kodensha). After the operation, the guinea pigs were left as such for about 5 minutes, and after the base PIP thereof was stabilized, the test with them was started. 50 µg/kg acetylcholine (hereinafter referred to as ACh, by Sigma) was intravenously administered to each test animal to induce airway contraction therein. After the elevated PIP was lowered to the base line thereof, ACh of the same concentration was again intravenously administered. This operation was repeated until the PIP increase (ΔPIP) was stabilized. After the ΔPIP stabilization was confirmed, ACh was intravenously administered (30 µg/kg or 40 µg/kg), and the value ΔPIP in this stage was referred to as a Pre value. 30 minutes thereafter, each test animal was made to inhale 0.1 µg/ml of LTD4 (by Funakoshi) for 30 seconds via an ultrasonic nebulizer (NE-U12, by Omron) , and after 15 minutes, ACh was again intravenously administered (30 µg/kg or 40 µg/kg) to each test animal to induce airway contraction therein. The value ΔPIP in this stage was rendered as a Post value. Relative to those of the LTD4 administration group, the animals of the control group were made to inhale saline in place of LTD4. The Post value and the Pre value were compared with each other in the control group and the LTD4 administration group, and the difference was an index of airway hypersensitiveness expression.

30 minutes before the start of the LTD 4 inhalation, a test substance was administered to the duodenum of each test animal. In place of the test substance, 0.5 % methyl cellulose (hereinafter abbreviated to MC) was administered to the animals of the control group relative to those of the test substance-administration group.

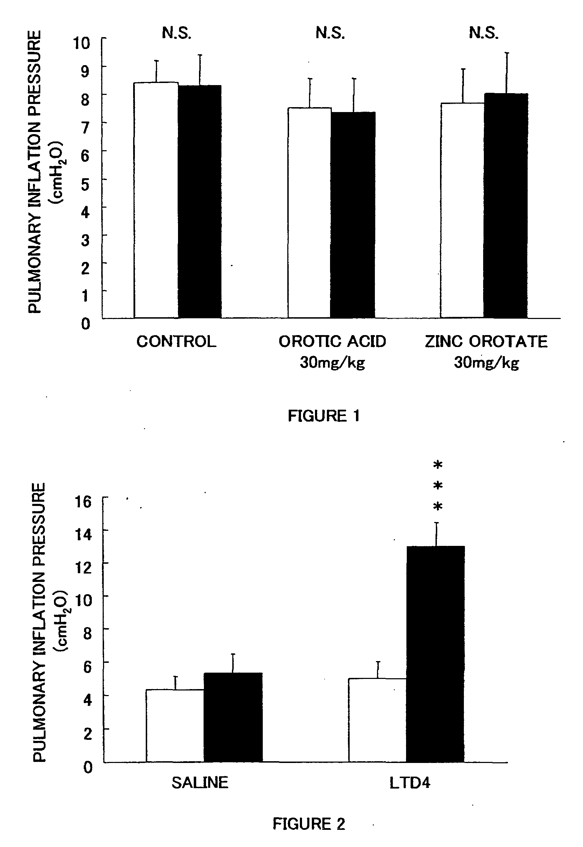

The data obtained were expressed as the mean value ± standard error thereof. A statistical analysis soft SAS was used for significance test. For significance test of two groups, pairedt-test was carried out (Fig. 1, Fig. 2) ; and for comparison test of multiple groups, Bartlet test was followed by 1-wayANOVA and Dunnett's multiple comparison test. In every test, p < 0.05 indicates a significant difference.

### 1. Preliminary Test:

The PIP increase (Pre value, white column) induced by the intravenous ACh administration (40 µg/kg) was 8.40 ± 0.76 cmH₂O; while the PIP increase (Post value, black column) after the inhalation of saline, as used for a solvent for LTD4 was 8.30 ± 1. 07 cmH₂O. There was found no significant difference between the two (Fig. 1).

Further, in case where orotic acid (30 mg/kg) or zinc orotate (30 mg/kg) was administered to the duodenum prior to the saline inhalation, there was still found no significant difference between the Pre value (7.50 ± 1.04, 7.67 ± 1.20 cmH₂O) and the Post value (7.33 ± 1.20, 8.00 ± 1.44 cmH₂O) (Fig. 1). Orotic acid and zinc orotate each were suspended in 0.5 % MC just before use.

### 2. Effect of Orotic Acid and Zinc Orotate:

When the test animals were made to inhale 0.1 µg/ml of LTD4 for 30 seconds, then the Pre value thereof induced by the intravenous administration of ACh (30 µg/kg) thereto was 5.00 ± 1.00 cmH₂O, and the Post value thereof significantly increased to be 13.00 ± 1.43 cmH₂O. This indicated the airway hypersensitiveness of the test animals (Fig. 2).

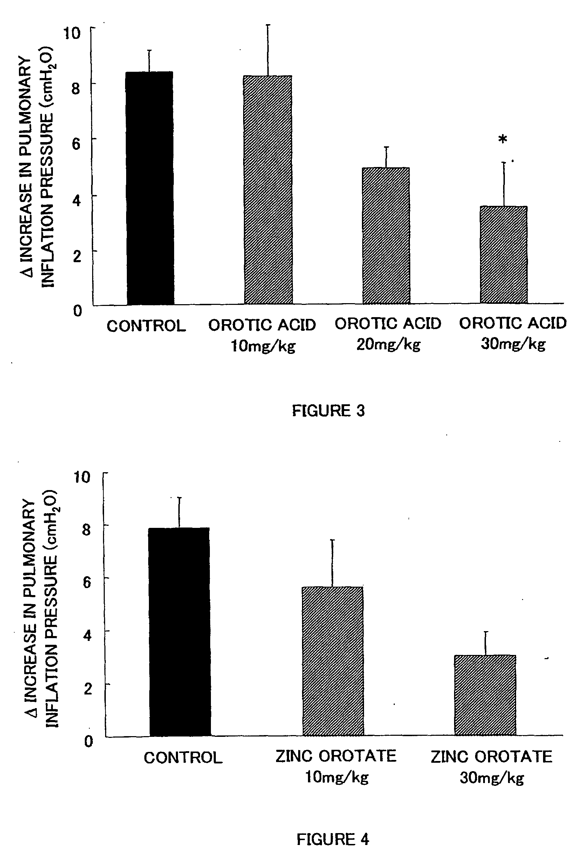

The dose-dependent effect of orotic acid against airway hypersensitiveness induced by LTD4 was investigated (Fig. 3). The vertical axis of the graph shows the increment in Pre value and Post value of ΔPIP.

As compared with those of the control group with no orotic acid administration thereto, the test animals with orotic acid administration (10 to 30 mg/kg) to the duodenum thereof showed airway hypersensitiveness inhibition that depends on the increase in the dose of the acid thereto. The dose of 30 mg/kg of the acid showed a statistical significance. ID50 of the acid was 20.1 mg/kg (Fig. 3).

The effect of zinc orotate was investigated. As compared with those of the control group with no zinc orotate administration thereto, the test animals with zinc orotate administration (10, 30 mg/kg) to the duodenum thereof showed airway hypersensitiveness inhibition that depends on the increase in the dose of the salt thereto (Fig. 4). ID50 of the salt calculated from the values of the two dose cases was 16.2 mg/kg.

The above confirms that intraduodenal administration of orotic acid or zinc orotate inhibits the LTD4-induced airway hypersensitiveness expression in guinea pigs.

Substances having an effect of tracheal smooth muscle relaxation and substances having an anticholinergic effect inhibit ACh-induced airway contraction. However, orotic acid did not have any influence on airway contraction, as in Fig. 1. Accordingly, it was seen that the airway hypersensitiveness inhibition by orotic acid or zinc orotate shown in Fig. 3 or Fig. 4 was not based on its effect for tracheal smooth muscle relaxation or anticholinergy. ID50 of orotic acid and zinc orotate is 20.1 and 16.2 mg/kg (in intraduodenal administration), respectively, and it was considered that the potency of the two, acid and salt to the animals tested herein was almost on the same level.

### Test Example 2 - Antiinflammatory Effect in Airway:

This was to confirm the presence or absence of antiasthmatic effect of orotic acid and zinc orotate. Specifically, the antiinflammatory effect of orotic acid and zinc orotate in airway was investigated.

The present test was carried out according to the method mentioned below, with reference to the method of Lagente et al [*Clin. Exp. Allergy,* 23, 1002-1010 (1993)] and the method of Dunn et al [*Am. Rev. Resp. Dis*., 137, 541-547 (1998)].

Hartley male guinea pigs (from Nippon Charles River) were raised at a constant temperature (22 to 23°C) and a constant humidity (50 to 60 %), and healthy animals of those having a body weight of from 350 to 450 g were used in the test. The guinea pigs were put in a plastic box (30 × 50 × 30 cm), and made to inhale 2 mg/ml of a solution of ovalbumin (hereinafter sometimes abbreviated to OA, from Sigma Aldrich) for 30 minutes, twice at an interval of 48 hours for passive sensitization. An ultrasonic nebulizer (NE-U12, by Omron) was used for the inhalation. Antigen induction in the test animals was carried out 13 to 15 days after the final sensitization, in the same plastic box as that for the sensitization. For it, the test animals were made to inhale 1 % (w/v) OA solution for 15 minutes to induce reaction. Those of the control group with no OA inhalation were made to inhale saline.

In this stage, a histamine H1-receptor antagonist, pyrilamine (by Sigma Aldrich) was intravenously administered (2 mg/kg) to the test animals 30 minutes before the antigen induction, for evading animal death owing to anaphylactic shock. Orotic acid, zinc orotate and a PDE4 inhibitor (rolipram, obtained through chemical synthesis) were orally administered one hour before the antigen induction. To those of the control group not administered with orotic acid, zinc orotate or rolipram, 0.5 % (w/v) MC solution was administered.

24 to 72 hours after the antigen induction, the bronchovesicles of the test animals were washed (BAL). Namely, the sensitized guinea pigs were anesthetized with pentobarbital (intravenous administration, 100 mg/kg), and 5 ml of saline was injected into the guinea pigs via a tracheal cannula through adisposable syringe (byTerumo). In about 15 seconds, the saline injected into the guinea pigs was recovered. This operation was repeated twice to collect the bronchoves icular washing fluid (BALF). BALF was centrifuged at 2500 rpm (1250 × g) at 4°C for 10 minutes. The supernatant was removed by suction, and the deposited cells were suspended in one ml of saline. In the suspension, the number of all leucocytes was counted with an automatic cell counter (Celltec MEK-4300, by Nippon Kodensha). A part of the cells were formed into a smear with Cytospin III (by Shandon Southern Instruments). The smear was stained with a Wright-Giemsa stain, and 500 cells therein were observed with an optical microscope. Specifically, eosinophils (Eos), neutrophils (Neu), macrophages (Mac), lymphocytes (Lym) and other cells (Other) were counted, and their ratio was calculated. The number of the respective cells was calculated by multiplying the number of all leucocytes by the ratio of the cells counted in the fraction.

The data obtained were expressed as the mean value ± standard error thereof. A statistical analysis soft SAS was used for significance test. For significance test of the two groups, Student's t-test was carried out; and for comparison test of multiple groups, Bartlet test was followed by 1-way ANOVA and Dunnett's multiple comparison test. In every test, p < 0.05 was regarded as a significant difference.

### 1. Preliminary Test relating to the number of cells having been invaded in BALF in 24, 48 and 72 hours after antigen induction:

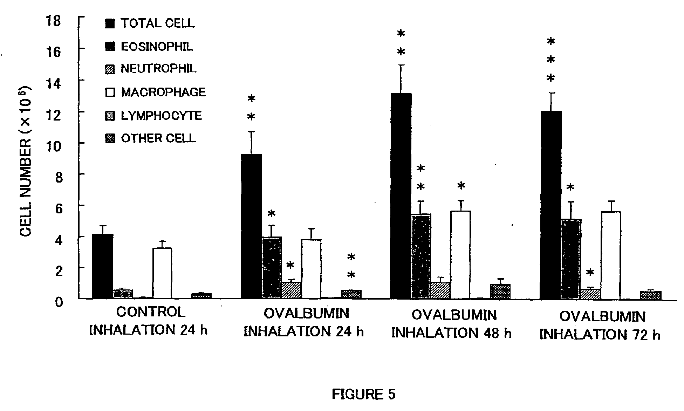

Significant increase in the number of the total cells and the number of eosinophilic leucocytes in BALF was admitted in any time of 24, 48 and 72 hours after the antigen induction in the test animals of the group having inhaled OA, as compared with those of the control group having inhaled saline (Fig. 5). In addition, 24 and 48 hours after the antigen induction, significant increase in the number of neutrophils was admitted (Fig. 5). The following experiment was conducted to investigate the effect of orotic acid and zinc orotate against the invasion of eosinophils and neutrophils relative to the number of the total cells.

The effect of the PDE4 inhibitor rolipram against cell invasion in 24 and 48 hours after the antigen induction was investigated, but any signif icant dif ference inthe cell invasion was not found between 24 hours and 48 hours (not illustrated). In the following experiment, therefore, the effect of orotic acid or zinc orotate against cell invasion in 24 hours after the antigen induction was investigated.

### 2. Effect of Orotic Acid and Zinc Orotate against Cell Invasion in 24 hours after antigen induction:

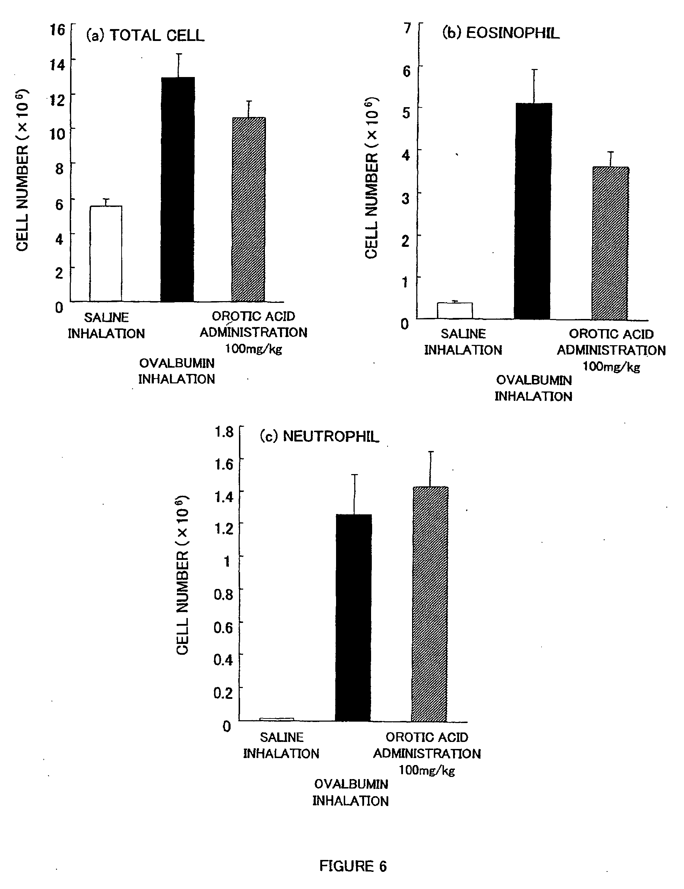

According to the process mentioned above, 100 mg/kg orotic acid was orally administered 1 hour before the antigen induction with OA so as to investigate the cell invasion in 24 hours after the antigen induction. The number of the total cells invaded and the number of eosinophils invaded both decreased in the group administered with orotic acid, as compared with that not administered with orotic acid (Fig. 6).

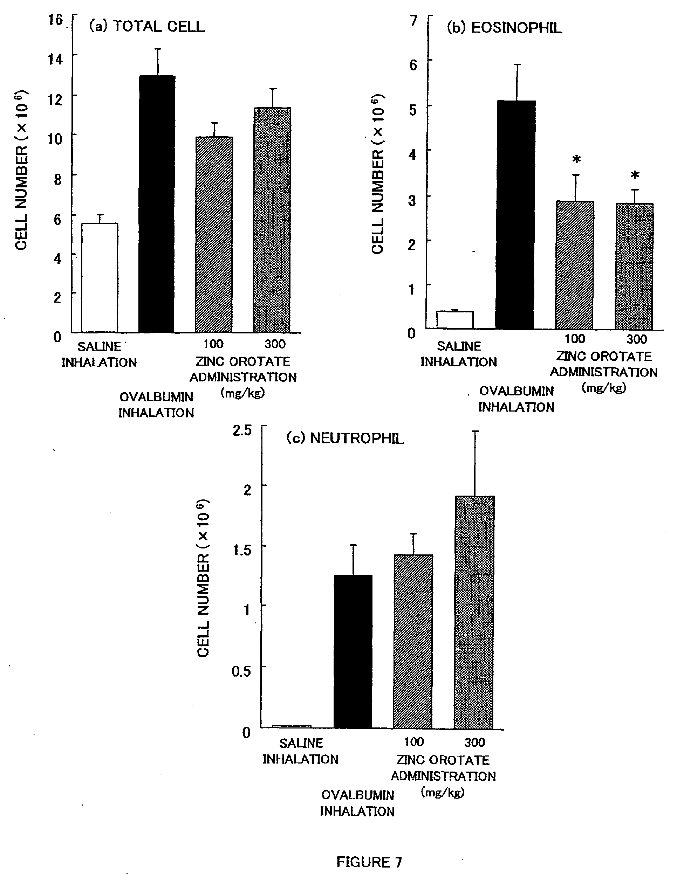

100 or 300 mg/kg of zinc orotate was orally administered 1 hour before the antigen induction with OA so as to make the same investigation as above. The number of the total cells invaded and the number of eosinophils invaded both decreased in the group administered with the different dose of zinc orotate (Fig. 7).

It was indicated that zinc orotate is useful for inflammatory airway diseases such as asthma.

### BRIEF DESCRIPTION OF THE DRAWINGS

Fig. 1 shows the result of investigation of the influence of orotic acid or zinc orotate administration on the acetylcholine-induced pulmonary inflation pressure change in guinea pigs under anesthetization, before inhalation of saline (white) and after inhalation thereof (black).

Orotic acid, zinc orotate (30 mg/kg) or 0.5 % MC was intraduodenally administered 30 minutes before the inhalation of saline. The data obtained are expressed as the mean value ± standard error of three cases. N.S. (not significant) indicates no statistically significant difference.

Fig. 2 shows the comparative result of acetylcholine-induced pulmonary inflation pressure change before inhalation of saline and leukotriene D4 (LTD4) (white) and after inhalation thereof (black).

In every group, 0.5% MC was intraduodenally administered 30 minutes before the inhalation of saline or LTD4. The data obtained are expressed as the mean value ± standard error of 5 or 6 cases. The significance test was effected through comparison of the pulmonary inflation pressure change before and after the inhalation. *** indicates significant difference with p < 0.001.

Fig. 3 shows the result of investigation of the effect of orotic acid to LTD4 inhalation-induced airway hypersensitiveness.

Δ pulmonary inflation pressure increase indicates the difference obtained by detracting the pulmonary inflation pressure change before inhalation of LTD4 from the pulmonary inflation pressure change after inhalation thereof. The data obtained are expressed as the mean value ± standard error of 5 cases. The significance test was effected through comparison with the 0.5 % MC administration group (control). * indicates significant difference with p < 0.05.

Fig. 4 shows the result of investigation of the effect of zinc orotate against LTD4 inhalation-induced airway hypersensitiveness.

The data obtained are expressed as the mean value ± standard error of 5 to 8 cases. The significance test is effected through comparison with the 0.5 % MC administration group (control).

Fig. 5 shows the result of investigating distribution of various cells in bronchovesicular washing fluid from ovalbumin (OA)-sensitized guinea pigs, 24, 48 and72 hours after inhalation of saline or OA. The columns indicate the total cells, eosinophils, neutrophils, macrophages, lymphocytes and other cells from the left-handed side. The vertical axis indicate the number of cells.

The data obtained are expressed as the mean value ± standard error of 4 or 5 cases. The significance test was effected through comparison with the saline administration group (control). *, ** and *** each indicate significant difference with p < 0.05, p < 0.01 and p < 0.001, respectively.

Fig. 6 shows the result of investigating the influence of orotic acid (100 mg/kg) on (a) the number of the total cells, (b) eosinophils invaded and (c) neutrophils invaded in bronchovesicular washing fluid from ovalbumin (OA) -sensitized guinea pigs, 24 hours after inhalation of OA.

Orotic acid was orally administered 1 hour before the OA inhalation. The data obtained are expressed as the mean value ± standard error of 9 or 10 cases.

Fig. 7 shows the result of investigating the influence of zinc orotate (100, 300 mg/kg) on (a) the number of the total cells, (b) eosinophils invaded and (c) neutrophils invaded in bronchovesicular washing fluid from ovalbumin (OA)-sensitized guinea pigs, 24 hours after inhalation of OA. Zinc orotate was orally administered 1 hour before the OA inhalation. The data obtained are expressed as the mean value ± standard error of 9 or 10 cases. The significance test was effected through comparison with the OA inhalation group. * indicates significant difference with p < 0.05.

### BEST MODES OF CARRYING OUT THE INVENTION

### Example 1:

The components are mixed according to the formulation mentioned below, and tabletted by the use of a tabletting machine (Hata Seisakusho, HT-AP15SS-U Model) into tablets having a diameter of 8 mm and a weight of 200 mg.

**Table 1**

| Components | Blend Ratio (wt.%) |
|---|---|
| Orotic Acid | 20 |
| Lactose | 40 |
| Calcium Lactate | 10 |
| Magnesium Stearate | 25 |
| Calcium Carbonate | 5 |

### Example 2:

The components are mixed according to the formulation mentioned below, and tabletted by the use of a tabletting machine (Hata Seisakusho, HT-AP15SS-U Model) into tablets having a diameter of 8 mm and a weight of 200 mg.

**Table 2**

| Components | Blend Ratio (wt.%) |
|---|---|
| Orotic Acid | 40 |
| Lactose | 20 |
| Calcium Lactate | 10 |
| Magnesium Stearate | 25 |
| Calcium Carbonate | 5 |

### Example 3:

The components are mixed according to the formulation mentioned below, and tabletted by the use of a tabletting machine (Hata Seisakusho, HT-AP15SS-U Model) into tablets having a diameter of 8 mm and a weight of 200 mg.

**Table 3**

| Components | Blend Ratio (wt.%) |
|---|---|
| Orotic Acid | 30 |
| Menthol | 10 |
| Lactose | 15 |
| Calcium Lactate | 10 |
| Magnesium Stearate | 25 |
| Calcium Carbonate | 5 |

### Example 4:

The components are mixed according to the formulation mentioned below, and tabletted by the use of a tabletting machine (Hata Seisakusho, HT-AP15SS-U Model) into tablets having a diameter of 8 mm and a weight of 200 mg.

**Table 4**

| Components | Blend Ratio (wt.%) |
|---|---|
| Orotic Acid | 20 |
| Herb | 20 |
| Lactose | 20 |
| Calcium Lactate | 10 |
| Magnesium Stearate | 25 |
| Calcium Carbonate | 5 |

### Example 5:

A dog food is produced according to the formulation mentioned below.

**Table 5**

| Components | Blend Ratio (wt.%) |
|---|---|
| Orotic Acid | 0.5 |
| Meat Meal | 35.0 |
| Corn Starch | 40.0 |
| Chicken Extract | 5.0 |
| Yeast Extract | 5.0 |
| Vegetable Oil and Fat | 5.0 |
| Calcium Lactate | 1.0 |
| Sodium Chloride | 1.0 |
| Sodium Hydrogenphosphate | 0.5 |
| Magnesium Carbonate | 0.5 |
| Iron Sulfate | 0.1 |
| Vitamin B₁ | 0.0005 |
| Vitamin B₂ | 0.0005 |
| Vitamin E | 0.001 |
| Niacin | 0.005 |
| Vitamin A | 2000 IU |
| Vitamin D | 150 IU |
| Water | 6.3 |

### Example 6:

A drink is produced according to the formulation mentioned below.

**Table 6**

| Components | Amount (g) |
|---|---|
| Orotic Acid | 0.5 |
| Pinedex #3 (Matsuya Chemical) | 49 |
| Ferric Pyrophosphate (iron source) | 0.1 |
| Phoscal EF | 1.0 |
| (calcium source, Nikko Fine Products) | |
| Vitamin Mix (Merck) | 1.0 |

### Example 7:

A refreshing drink (1000 ml for 10 bottles) is produced according to the formulation mentioned below.

**Table 7**

| Components | Amount (g) |
|---|---|
| Orotic Acid | 0.3 |
| Vitamin C | 1 |
| Vitamin B1 | 0.005 |
| Vitamin B2 | 0.01 |
| Vitamin B6 | 0.025 |
| Liquid Sugar | 150 |
| Citric Acid | 3 |
| Flavor | 1 |

### Example 8:

A tea drink (1000 ml) is produced according to the formulation mentioned below.

**Table 8**

| Components | Amount (g) |
|---|---|
| Orotic Acid | 0.3 |
| Tea Leaves | 15 |

### Example 9:

A cookie dough (for 30 cookies) is produced in a known manner according to the formulation mentioned below.

**Table 9**

| Components | Amount (g) |
|---|---|
| Orotic Acid | 20 |
| Herb | 10 |
| Flour of low gluten | 10 |
| Starch | 100 |
| Water | 74 |
| Baking Powder | 14 |
| Salt | 2 tea spoons |
| Egg | 1/2 tea spoon |
| Butter | one |
| Milk | 80 g |
| Honey | 2 table spoon |
| | a little |

### Example 10:

A bread dough (for 4 loaves) is produced in a known manner according to the formulation mentioned below.

**Table 10**

| Components | Amount (g) |
|---|---|
| Orotic Acid | 15 |
| Vitamin Mixture | 15 |
| Flour of high gluten | 1000 |
| Sugar | 50 |
| Salt | 20 |
| Skim Milk | 20 |
| Shortening | 60 |
| Yeast (raw) | 30 |
| Yeast Food | 1 |
| Water | 650 |

### Example 11:

A chewing gum paste (for 30 sticks) is produced in a known manner according to the formulation mentioned below.

**Table 11**

| Components | Amount (g) |
|---|---|
| Orotic Acid | 1 |
| Gum Base | 25 |
| Sugar | 63 |
| Glutinous Starch Syrup | 10 |
| Flavor | 1 |

### Example 12:

A candy paste (for 20 candies) is produced in a known manner according to the formulation mentioned below.

**Table 12**

| Components | Amount (g) |
|---|---|
| Orotic Acid | 1 |
| Sugar | 80 |
| Glutinous Starch Syrup | 20 |
| Flavor | 0.1 |

### Example 13:

A marmalade is produced in a known manner according to the formulation mentioned below.

**Table 13**

| Components | Amount (g) |
|---|---|
| Orotic Acid | 5 |
| Herb | 5 |
| Skin of Watson Pomelo | 500 |
| Sugar | 200 |
| Juice of Watson Pomelo | from one Watson pomelo |

### Example 14:

Hard capsules (360 mg/capsule) are produced according to the formulation mentioned below.

**Table 14**

| Components | Amount (mg) |
|---|---|
| Orotic Acid | 250 |
| Lactose | 60 |
| Corn Starch | 30 |
| Hydroxypropyl Cellulose | 20 |

60 mg of lactose and 30 mg of corn starch are added to 250 mg of orotic acid, and mixed. Aqueous solution of 20 mg of hydroxypropyl cellulose is added thereto and kneaded. Next, the mixture is granulated through an extrusion granulator into granules in a known manner. The granules are encapsulated in hard gelatin to prepare hard capsules.

### Example 15:

Soft capsules (220 mg/capsule) are produced according to the formulation mentioned below.

**Table 15**

| Components | Amount (mg) |
|---|---|
| Orotic Acid | 50 |
| Soybean Oil | 170 |

50 mg of orotic acid is added to 170 mg of soybean oil, and mixed. Next, using a rotary die-type automatic encapsulator, the mixture is encapsulated in soft gelatin to prepare soft capsules in a known manner.

### Example 16 - Preparation of Orotic Acid-Containing Drops:

Orotic acid-containing drops are produced according to the formulation mentioned below.

**Table 16**

| Components | Amount (kg) |
|---|---|
| Caramel Base | |
| Sugar (mid grain size) | 35.0 |
| Corn Syrup 43° Baume | 21.0 |
| Chemical Mixture | |
| Polyethylene Glycol | 2.75 |
| (molecular weight 6,000) | |
| Orotic Acid | 5.0 |
| Citric Acid | 60.0 |
| Wild Cherry-Like Flavor | 0.06 |

Sugar is dissolved in 5.5 liter of water, and glucose-containing corn syrup is added thereto and well mixed. In this stage, any desired dye may be added thereto to color it in any desired manner. The dye well soluble is used.

The mixture is put into a steam jacket kettle heated at 125°C, and the mixture is transferred into a storage container via a pump, and then fed into a continuous cooker.

While the syrup passes through the coil in the cooker, the temperature of the syrup reaches from 125 to 150°C. Next, this is fed into a receiving kettle kept at a vacuum degree of 28 to 29 inch-Hg, via a steam vacuum ejector over a period of about 6 to 7 minutes. During this, water is removed until the water content of it reaches about 1 % or less, and a caramel base melt is thus formed. Next, this caramel base is gradually cooled.

Orotic acid, citric acid and wild cherry-like flavor (powder) are added to polyethylene glycol, and the resulting mixture is heated at about 90°C to be fluidized.

The thus-obtained hot fluid mixture is rapidly added to the caramel base melt (cooled to about 100°C or slightly lower than it) while suitably mixed. Next, the whole mass is well mixed, then transferred into a spinning machine, and extruded through a lozenge-shaping die. Alternatively, the caramel base melt with orotic acid added thereto is cast onto a cooling table, on which it is semi-solidified and then shaped into any desired form for unit dose administration of the chemical.

### INDUSTRIAL APPLICABILITY

The present invention provides antiinflammatory agents for airway, foods and drinks, animal feeds, food additives and feedadditives, each comprising, as the active ingredient thereof, orotic acid or its derivative or a salt thereof; and a method for preventing or treating airway inflammations which comprises administering orotic acid or its derivative or a salt thereof.

## Claims

1. An antiinflammatory agent for airway, which comprises, as the active ingredient thereof, orotic acid or its derivative or a salt thereof.

2. The antiinflammatory agent for airway as claimed in claim 1, which has an antitussive effect.

3. The antiinflammatory agent for airway as claimed in claim 1 or 2, which is against airway inflammations selected from those caused by allergic asthma, those caused by pollinosis and those caused by bronchitis.

4. A food and drink or an animal feed, which comprises orotic acid or its derivative or a salt thereof.

5. A food additive or a feed additive, which comprises orotic acid or its derivative or a salt thereof.

6. A method for preventing or treating human or non-human animal airway inflammations, which comprises administering orotic acid or its derivative or a salt thereof.

7. Use of orotic acid or its derivative or a salt thereof for the manufacture of antiinflammatory agents for airway.
